# EUROPÄISCHE PATENTANMELDUNG

(11) **EP 0 920 833 A1**
(43) Veröffentlichungstag der Anmeldung: **09.06.1999**
(21) Anmeldenummer: 98123118.6
(22) Anmeldetag: 04.12.1998
(51) Int. Cl.: A61B 8/12, A61B 8/08, A61B 5/103

(54) **sonographisches Elastographiesystem**

(30) Priorität: 05.12.1997 DE 19754085
(71) Anmelder: Ermert, Helmut, Institut für Hochfrequenztechnik, 44780 Bochum (DE); Lorenz, Andreas, 44135 Dortmund (DE); Wiebe, Peter, 85285 Gevelsberg (DE)
(72) Erfinder: Ermert, Helmut, Institut für Hochfrequenztechnik, 44780 Bochum (DE); Lorenz, Andreas, 44135 Dortmund (DE); Wiebe, Peter, 85285 Gevelsberg (DE)

(57) **Zusammenfassung**

Die mechanischen Eigenschaften von biologischem Gewebe (z.B. Elastizitätsparameter wie der Elastizitätsmodul) sind für die Beurteilung des Zustandes des Gewebes von großem Interesse. In der medizinischen Diagnostik deuten Veränderungen der Elastizitätseigenschaften auf histologische und u.U. pathologische Veränderungen hin; z. B. zeichnen sich Tumoren meist durch die Bildung von Geschwülsten und Verhärtungen ( 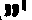 Knoten") aus, die manuell tastbar sind.

Die Aufgabe der Erfindung ist es, Ultraschallbilder bei vorgegebenen Kompressionsstufen aufzunehmen bzw. den Kompressionsdruck während der Aufnahme zu protokollieren, um eine Anzeige der elastischen Eigenschaften von Körpergewebe effizienter zu gestalten und durch die genaue Messung und Kontrolle des applizierten Druckes eine quantitative Rekonstruktion des Elastizitätsmoduls zu unterstützen.

## Beschreibung

### Kurzbezeichnung: Ein sonographisches Elastographiesystem

Die Erfindung betrifft eine Meßeinrichtung entsprechend dem Oberbegriff des Anspruchs 1.

### Anwendungsgebiet

Die mechanischen Eigenschaften von biologischem Gewebe (z.B. Elastizitätsparameter wie der Elastizitätsmodul) sind für die Beurteilung des Zustandes des Gewebes von großem Interesse. In der medizinischen Diagnostik deuten Veränderungen der Elastizitätseigenschaften auf histologische und u.U. pathologische Veränderungen hin; z. B. zeichnen sich Tumoren meist durch die Bildung von Geschwülsten und Verhärtungen ( 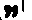 Knoten") aus, die manuell tastbar sind.

Die Aufgabe der Erfindung ist es, Ultraschallbilder bei vorgegebenen Kompressionsstufen aufzunehmen bzw. den Kompressionsdruck während der Aufnahme zu protokollieren, um eine Anzeige der elastischen Eigenschaften von Körpergewebe effizienter zu machen und durch genaue Messung und Kontrolle des applizierten Druckes eine quantitative Rekonstruktion des Elastizitätsmoduls zu unterstützen.

### Stand der Technik

Die sogenannte Tastbefundung ist ungenau und unempfindlich (schlechte räumliche Auflösung). Wesentlich besser ist in dieser Beziehung die sogenannte Elastographie, bei der elastische Gewebeeigenschaften technisch erfaßt und z.B. in Form von Schnittbildern qualitativ oder quantitativ visualisiert werden. Dabei bedient man sich hauptsächlich des Ultraschalls, wie er als bildgebendes Verfahren in der medizinischen Diagnostik angewandt wird. In zeitlich nacheinander aufgenommenen Ultraschallbildern können geringste Verschiebungen oder Verformungen innerhalb der dargestellten Gewebestruktur durch die Auswertung von Bildsequenzen erfaßt und ausgewertet werden. Wird auf einen Gewebebereich ein mechanischer Druck ausgeübt, der eine Verformung des Gewebes zur Folge hat, so verformen sich Bereiche mit unterschiedlichen Elastizitätseigenschaften verschiedenartig. Das Elastographiesystem wertet diese Verformungen durch den numerischen Vergleich der Einzelbilder aus und stellt die unterschiedlichen Elastizitätsparameter im Bild dar. Die notwendige Kompression des Gewebes, die z.B. extern provoziert wird, ist nur gering und beträgt bei Anwendung des üblichen diagnostischen Ultraschalls nur Bruchteile von Millimetern. Wichtig ist dabei eine quantitative Kontrolle des Kompressionsdruckes, der z. B. zur Bildauswahl und zur Rekonstruktion der Elastizitätsverteilung eingesetzt werden kann.

Ein Verfahren der Ultraschall-Elastographie von Körpergewebe ist erstmalig in einem Aufsatz von J. Ophir et al im Jahre 1991 [1], [2] beschrieben worden. Dabei werden Ultraschallbilder bzw. die korrespondierenden hochfrequenten Ultraschallechosignale so ausgewertet, daß Verschiebungen des Körpergewebes Ultraschallbilder bzw. die korrespondierenden hochfrequenten Ultraschallechosignale so ausgewertet, daß Verschiebungen des Körpergewebes zwischen zwei, mit verschiedener Kompression aufgenommenen Gewebebildern berechnet werden. Auf diese Weise lassen sich die bereits erläuterten Rückschlüsse auf die Elastizität des Organs mit Hilfe von Dehnungsbildern gewinnen. Eine quantitative Rekonstruktion des Elastizitätsmoduls ist jedoch ohne die Kenntnisse der Randbedingungen, d.h. des applizierten Druckes nicht möglich.

In der Literatur werden verschiedene Ansätze vorgestellt, mit denen die Abbildungseigenschaften eines Elastographie-Systems verbessert werden können. Vielversprechend im Hinblick auf das Signal-Rauschverhältnis und den Kontrast der Elastographiebilder sind Ansätze, die mehrfache Kompressionsstufen des abzubildenden Gewebes auswerten [5]. Bei diesem Ansatz werden bis zu 120 Bilder in einer Aufnahmeserie aufgenommen und mit bekannten Methoden der Ultraschall-Elastographie [3], [4] weiterverarbeitet, es findet jedoch keine Aufzeichnung und Auswertung des Druckes der Sonde auf das Gewebe statt.

### Nachteile des Standes der Technik

Ein Nachteil der bisher publizierten bzw. praktizierten Verfahren ist, daß zu keiner Zeit die Kraft auf das Gewebe bekannt ist, die wertvolle Informationen bei einer quantitativen Rekonstruktion des Elastizitätsmoduls liefern kann. Außerdem ist ein solcher Ansatz speicher-, rechen- und damit kostenintensiv, da Auswahlkriterien dazu herangezogen werden müssen, optimale Bildfolgen aus den aufgenommen Daten auszuwählen und weiterzuverarbeiten.

### Aufgabe der Erfindung

Die Aufgabe der Erfindung ist es, Ultraschallbilder in vorgegebenen Kompressionsstufen aufzunehmen bzw. den Kompressionsdruck während der Aufnahme zu protokollieren, um die Aufnahme und Anzeige der elastischen Eigenschaften von Körpergewebe effizienter zu machen und durch genaue Messung und Kontrolle des applizierten Druckes eine quantitative Rekonstruktion des Elastizitätsmoduls zu unterstützen.

### Lösung der Aufgabe

Diese Aufgabe wird durch ein Meßsystem mit den Merkmalen des Anspruch 1 gelöst.

Die Kompression des Gewebes wird durch den Ultraschallwandler, mit dem die Ultraschallbilder aufgenommen werden, hervorgerufen. Die Kraft, die der Schallwandler auf das Gewebe ausübt, wird durch eine mit diesem Wandler verbundene Vorrichtung gemessen, und es werden Ultraschallbilder in vorgegebenen Kompressionsstufen aufgenommen. Insbesondere läßt sich bei endoskopischen Schallwandlern, (z. B. bei einer transrektalen oder transvaginalen Sonde), die für gesonderte Kraftmeßvorrichtungen nur wenig Platz erlauben, die Kraft mittels eines am Schaft der Schallsonde angebrachten Dehnungssensors nach dem Biegebalkenprinzip bestimmen. Für nicht-endoskopische Schallköpfe, d.h. Schallköpfe die auf der Körperoberfläche appliziert werden, kann eine hinsichtlich der Übergangseigenschaften des Schalls angepaßte, schalldurchlässige Kappe auf dem Schallkopf angebracht werden. Eine Kraftmessung ist dann mittels eines hydrostatischen Drucksensors möglich.

### Vorteile der Erfindung

Dieser Ansatz erlaubt eine Montage der Sensoren auf handelsübliche Ultraschallsonden mit nur geringfügiger Modifikation der Schallsonde. Es werden keine gerätebaulichen Veränderungen am Ultraschallgerät vorgenommen. Weiterhin erfolgt die Bildaufnahme zu gezielt vorgegebenen Kraft- bzw. Druckstufen, was den Speicher- und Rechenaufwand im Vergleich zu bekannten Ansätzen erheblich reduziert und/oder der Druck wird während der Messung kontinuierlich aufgezeichnet, um eine nachfolgende quantitative Rekonstruktion der elastischen Gewebeeigenschaften zu ermöglichen. Die Erfindung macht bei der Anwendung starrer endoskopischer Schallsonden eine quantitative Elastographie erst möglich.

### Ausführungsbeispiel

Ausführungszeichnungen der Erfindung sind in den Zeichnungen dargestellt und werden im folgenden am Beispiel einer endoskopischen Sonde näher beschrieben.

### Zu Skizze 1: Schematische Darstellung des Elastographie-Systems

Das Meßsystem besteht im wesentlichen aus drei Funktionseinheiten:
a) Ein handelsübliches Ultraschallsystem (Skizze 1 (14) im folgenden kurz 1.14) mit verschiedenen endoskopischen und nicht-endoskopischen Sonden (1.1+1.2). Am Ultraschallgerät muß dabei eine Möglichkeit zur Aufnahme von Video oder HF-Daten bestehen (1.11).
b) Eine Interfaceeinheit (in Skizze 1 mit *Elastographie-Interface* bezeichnet), zur optionalen Anzeige des Druckes und zur Umwandlung der analogen Sensorsignale in korrespondierende, diskrete Signale zur Verwendung der Signale in der Computereinheit (1.12)
c) Eine Computereinheit (1.12), die mit entsprechenden Peripheriegeräten zur Aufnahme der HF-Daten (1.11) ausgerüstet ist (z.B. A/D-Wandler- oder Framegrabberkarte), und zur Datenaufnahme und -weiterverarbeitung verwendet wird.

Kernstück der Erfindung ist die Einrichtung zur Messung der Kraft, die auf das Gewebe ausgeübt wird ( 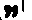 Elastographie-Interface"), und die Erzeugung eines mit der Computereinheit lesbaren Signals (1.15+1.10), daß den Betrag der Kraft in ein diskretes Signal umwandelt (A/D-Wandlung). Dieses Signals kann zur Extraktion eines Triggersignals (1.10) zur Bildaufnahme verwendet werden.
Dazu sind an der endoskopischen Sonde (1.1) ein oder mehrere Dehnungssensoren (1.3) angebracht. Ein Ausführungsbeispiel zu dieser Art der Kraftmessung ist in den Skizzen 2 und 3 dargestellt. An nicht-endoskopischen Sonden (1.2) wird eine wassergefüllte Kappe aus schalldurchlässigem Material angebracht. Der hydrostatische Wasserdruck in der Kappe kann dann mittels eines Drucksensors (1.4) gemessen werden. Ein Ausführungsbeispiel einer nicht-endoskopischen Sonde ist in der Skizze 4 dargestellt.
In der Skizze 1 ist ein Ausführungsbeispiel für das Elastographie-Interface skizziert.

Die Sensorsignale werden einer Meßeinheit (1.5) zugeführt. Üblicherweise wird hier eine Brückenschaltung eingesetzt, die bei kleinen Biegungen bzw. kleinen Druckänderungen eine zur Kraft proportionale Spannung (1.7) liefert. Um einen definierten Anfangszustand zu erzielen, wird die Meßeinheit abgeglichen (1.6). Das Ausgangssignal der Meßeinheit (1.7) kann einerseits zur Anzeige der Kraft verwendet werden (1.8), und wird andererseits einer Stufenkodierung (1.9) zugeführt. Diese Stufenkodierung erzeugt ein Triggersignal (1.10), welches zur Aufnahme der Bilddaten vom Video- oder HF-Ausgang (1.11) des Ultraschallgerätes (1.14) verwendet wird. Eine mögliche Realisierung der Stufenkodierung basiert auf der Verwendung eines A/D-Wandlers mit nachgeschaltetem BCD/Dezimal-Dekoder.
BCD/Dezimal-Dekoder des Typs 74xx148 verfügen über ein Priority-Flag, welches das Anliegen einer Stufe kennzeichnet. Dieses Flag läßt sich günstig zur Triggerung der Aufnahmeeinheit (1.12) verwenden. Gleichzeitig kann die Höhe der Stufe, ebenfalls an den Rechner weitergegeben werden (1.15). Die aufgenommenen Daten werden dann im Computer mit bekannten Algorithmen weiterverarbeitet, und zur Anzeige (1.13) gebracht.

### Zu Skizze 2 und Skizze 3: Endoskopische Schallsonde mit Dehnungssensor

Die Skizzen 2 und 3 enthalten Detailzeichnungen zur Anbringung der Dehnungssensoren am Sondenschaft, und erläutern die Kraftmessung nach dem Biegebalkenprinzip. Die Sonde wird am Griff (2.5) gehalten und bei ihrer Verwendung mit der Spitze (2.1) in eine Körperöffnung (in der Regel transrektal oder transvaginal) eingeführt. Die Bildaufnahme erfolgt dabei üblicherweise senkrecht zur Ausrichtung des Sondenschaftes in der Ebene B, parallel zur Richtung der wirkenden Kraft F. Zu einer auf das Gewebe wirkenden Kraft F resultiert eine Gegenkraft auf die Sonde, welches zu einer Biegung des Sondenschaftes (2.2) führt. Diese Biegung kann mit Hilfe der Dehnungssensoren (2.3) detektiert werden.
Dehnungssensoren gibt es in verschiedenen Ausführungsformen und können unterschiedlich, d.h. einzeln, paarweise, oder vierfach, in vom Hersteller angegebenen Positionen und Verschaltungen am Schaft der Sonde (2.4) befestigt werden. In der Skizze 3 wird die genaue Lage der zwei, in unserem Ausführungsbeispiel angebrachten Dehnungssensoren angedeutet.

### zu Skizze 4: Nicht-endoskopische Schallsonde mit hydrostatischem Drucksensor

Bei einer nicht-endoskopischen Sonde wird zur Kraftmessung eine wassergefüllte, schalldurchlässige Kappe (2) auf dem Schallwandler angebracht. Diese Kappe ist mit einer Metallasche (1) befestigt, und so abgedichtet, daß ein Auslaufen der Flüssigkeit verhindert wird. An einer Schlauchverlängerung (4), dessen Zugang zum Innenraum der Kappe ähnlich einem Fahrradventil ausgeführt werden kann (3), ist ein hydrostatischer Drucksensor (5) mit Zuleitungen (6) befestigt, der beliebig am Handgriff der Schallsonde (7) befestigt werden kann. Eine weitere Ausführungsform wäre, daß der Sensor direkt in die schalldurchlässige Kappe eingebaut wird. Eine auf den Wandler wirkende Kraft F führt zu einer Verformung der Kappe, und damit zu einer Veränderung des Druckes innerhalb des geschlossenen, mit Wasser gefüllten Systems. Diese Druckänderung kann mit Hilfe des Drucksensors gemessen werden.

### Literaturangaben:

[1]Ophir J., Céspedes I., Ponnekanti H., Yazdi Y., Li X.: Elastography: A quantitative method for imaging the elasticity of biological tissues. *Ultrasonic Imaging 13*, 111-114, 1991
[2]Céspedes I., Ophir J., Ponnekanti H., Maklad N.: Elastography: Elasticity imaging using ultrasound with application to muscle and breast imaging in vivo. *Ultrasonic Imaging 15*, 73-88, 1993
[3]O'Donnell M., Skovoroda A. R., Shapo B. M., Emilianov S. Y.: Internal displacement and strain imaging using ultrasonic speckle tracking. *IEEE transactions on ultrasonics ferroelectrics and frequency control*, 41, 314-325, May 1994
[4]Lubinski M. A., Emilianov S. Y., Raghavan K. R.: Lateral displacement estimation using tissue incompressibility. *IEEE transactions on ultrasonics ferroelectrics and frequency control*, 43, 234-246, 1995
[5]Emilianov S. Y., Lubinski M. A., Skovoroda A. R., Erkamp R. Q., Leavey S. F., Wiggins R. C., O'Donnell M.: Reconstructive ultrasound elasticity imaging for renal pathology detection. Proc. 1997 IEEE *Ultrasonics Symposium*, IEEE Press, Piscataway, MD, USA.

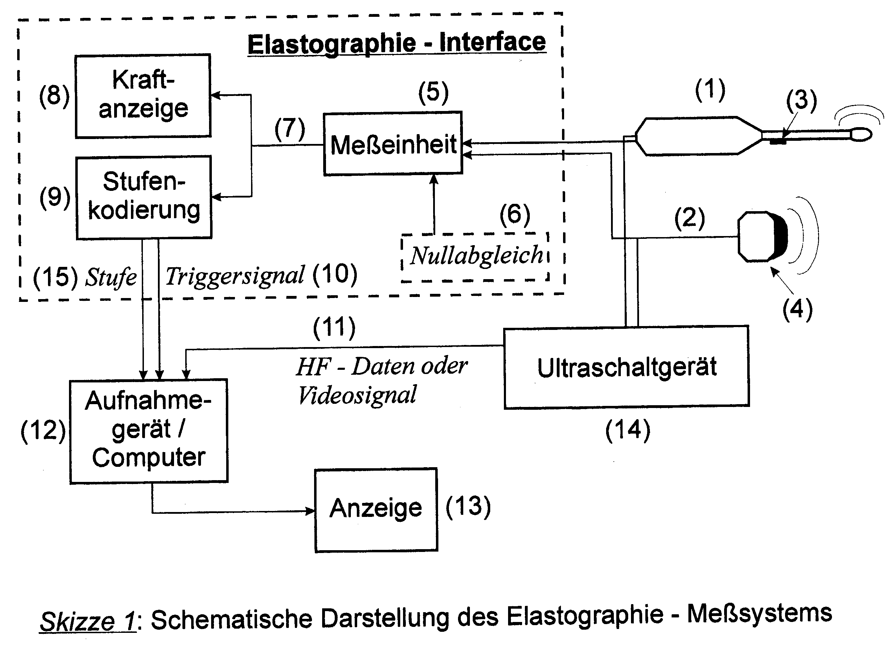

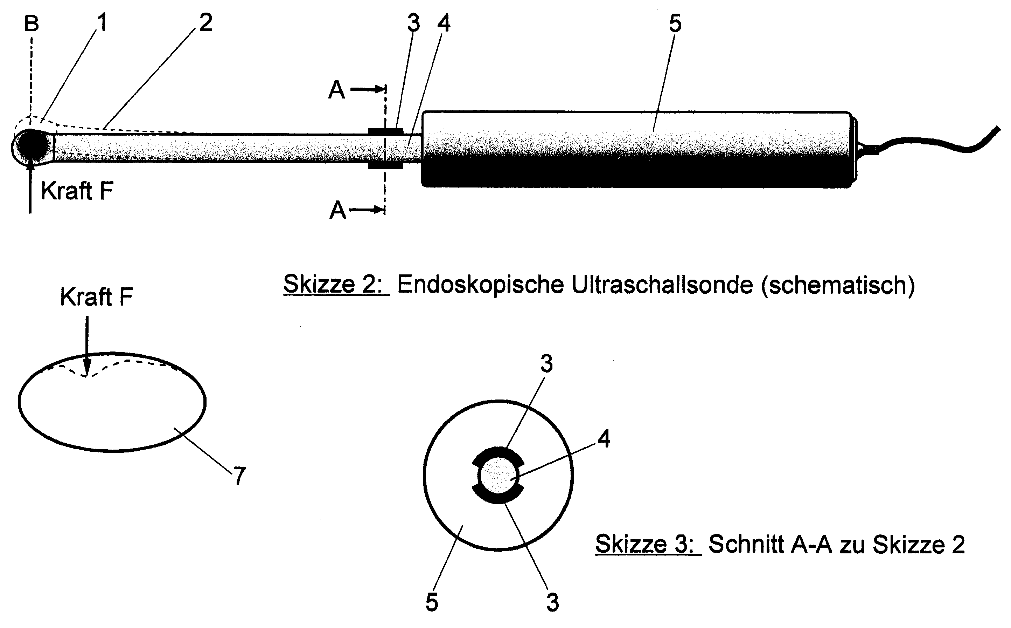

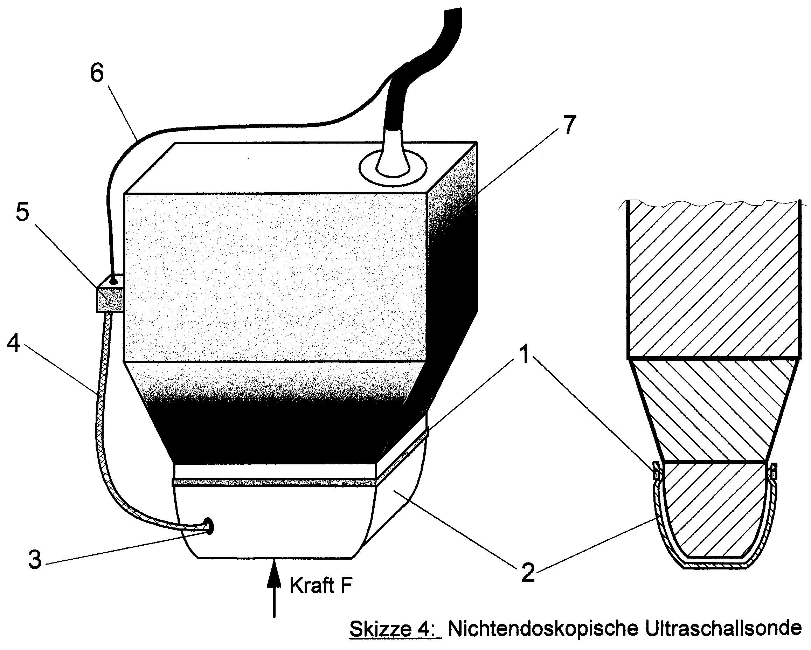

## Patentansprüche

1. Ein Meßsystem zur Bestimmung und Visualisierung von elastischen Gewebeeigenschaften mit diagnostischem Ultraschall, **dadurch gekennzeichnet, daß** die Kompression des Gewebes durch die Ultraschallsonde bewirkt wird und die von der Sonde auf das Gewebe ausgeübte Kraft gemessen, kontrolliert und dazu verwendet wird, Ultraschallbilder bzw. Bildsequenzen bei vorher festgelegten Kompressionsstufen aufzunehmen (Skizze 1).

2. Ein Meßsystem nach Anspruch 1, **dadurch gekennzeichnet, daß** bei entsprechend gestalteten Ultraschallwandlern (starre Endoskopiesonden) die Kraft auf das Gewebe mittels eines mit dem Schallwandler verbundenen Dehnungssensors nach dem Biegebalkenprinzip gemessen wird (Skizzen 2 und 3).

3. Ein Meßsystem nach Anspruch 1, **dadurch gekennzeichnet, daß** die Kraft auf das Gewebe mittels eines mit dem Schallwandler verbundenen hydrostatischen Drucksensors gemessen wird (Skizze 4).

4. Ein Meßsystem nach Anspruch 1, **dadurch gekennzeichnet, daß** die Messung der Kraft auf das Gewebe mit handelsüblichen Ultraschallgeräten bzw. Sonden mit geringfügiger Modifikation der Schallsonde erfolgen kann, ohne gerätebauliche Veränderungen am Ultraschallgerät vornehmen zu müssen.
